# NEW EUROPEAN PATENT SPECIFICATION

(11) **EP 3 145 466 B2**
(45) Date of publication and mention of the opposition decision: **11.01.2023**
(45) Mention of the grant of the patent: 20.06.2018
(21) Application number: 15727768.2
(22) Date of filing: 19.05.2015
(51) Int. Cl.: A61F 13/58, A44B 18/00, A61F 13/62

(54) **ROLLS FOR MANUFACTURE OF FASTENING TABS**
ROLLEN ZUR HERSTELLUNG VON BEFESTIGUNGSSCHELLEN
ROULEAUX DE FABRICATION DE LANGUETTES DE FIXATION

(30) Priority: 19.05.2014 EP 14168822
(43) Date of publication of application: 29.03.2017
(73) Proprietor: 3M Innovative Properties Company, St. Paul, MN 55133-3427 (US)
(72) Inventor: GABRIEL, Siegfried R., 41453 Neuss (DE); KITZER, Peter, 41453 Neuss (DE); PEIFFER, Andreas, 41453 Neuss (DE)
(74) Representative: Vollmers, Hans-Gerd
(86) International application number: PCT/US2015/031526
(87) International publication number: WO 2015/179368

(56) References cited:
- EP-A1- 2 679 112
- EP-A1- 2 679 112
- EP-A2- 2 110 110
- WO-A1-2007/072386
- WO-A1-2011/094474
- FR-A1- 2 767 653
- FR-A1- 2 767 653
- US-A- 4 592 118
- US-A1- 2007 173 781
- US-A1- 2007 173 781
- US-A1- 2012 042 483
- US-A1- 2012 042 483

## Description

The present invention relates to rolls of an elongated material for manufacture of fastening tabs as well as to methods of manufacturing fastening tabs.

Fastening tabs are widely used in hygiene industry. One important application of such fastening tabs relates to the closure system of diapers. These fastening tabs comprise a substrate having a manufacturer's end typically including an adhesive for attaching a tab to a disposable article, e.g., to the outer side of a diaper's ear, and an opposite user's end which may be gripped by a user and attached to a landing zone of, e.g., the diaper. The user's end typically comprises a mechanical fastening material, such as a hook material for releasably attaching the fastening tab to e.g. a corresponding loop material provided in the landing zone, as well as a finger lift, an area adjacent to the outer edge of the user's end free of fastening material for facilitating the gripping and lifting of the user's end by the user. Such fastening tabs are typically provided in large rolls of an elongated endless material, from which individual tabs are cut. Such fastening tabs are often provided on so-called twin rolls in the form of a planetary roll. The elongated material of such a twin roll comprises, along the cross or width direction, two adjacent fastening tabs, which are separated from one another through a center line of the elongated material. In these twin rolls, the user's ends, typically comprising the fastening material and the narrow finger lift, are arranged in the center of the elongated material with the finger lift areas being adjacent to one another along the center line, while the manufacturer's ends are provided at the outer regions of the elongated material, where adhesive then is applied near but spaced apart (∼2-3 mm) from the edges. The adhesive is applied away from the edge of the manufacturer's end, because during processing the web typically shakes in the cross direction along its path thus necessitating the positioning of the adhesive applicator head inwardly away from the edge.

These twin rolls according to the prior art have various disadvantages. The cross dimension of the elongated material (-90 mm) is much lower than the diameter of the roll (which may be up about 1 m) and since the thickest portion of the elongated material i.e., the mechanical fastening elements in the user's ends is provided towards the center of the elongated material with the low profile manufacturer's ends to the outer regions, these twin rolls exhibit roll instability. In addition, it has been observed that upon storage, dust and dirt particles may penetrate deep into the roll. Finally, due the gap between the edge and the adhesive in the manufacturer's end, after attaching the tab via the adhesive to the absorbent article the non-adherent part of the manufacturer's end protrudes from the final product like a flap and may create sharp edges or corners.

Surprisingly by providing a twin roll of an elongated material where the mechanical fastening elements are provided in the two outer longitudinal edge portions (each being at most 30% of the total width of the elongated material) and where on the same side at least the innermost central region (which for example may have a width up to 30% of the total width of the elongated material) of the central portion is free of said fastening elements so that such portion or at least a part of such portion thereof can be used to affix tab(s) obtained from such roll to absorbent article(s) (and thus accordingly the opposite side is free of adhesive), it is possible to provide a twin roll having improved roll stability and resistance to penetration of dirt and dust into the roll. In other words, by providing the user's ends with the fastening elements in the two outer edge regions at or close to the edges of the elongated material and the manufacturer's ends there between, the fastening elements, i.e. the thickest portions of the elongated material, are provided in the two outer edge portions at or close to the edges of the roll, thus facilitating roll stability and acting as a barrier helping to prevent or reduce ingress of dust and dirt particles into the roll.

Accordingly in a first aspect of the present invention there is provided a roll of an elongated material for manufacture of fastening tabs, the elongated material comprising first and second major surfaces, two longitudinal edge portions and a central portion provided in the width direction in between the two longitudinal edge portions, the width of each longitudinal edge portion being at most 30% of the total width of the elongated material, wherein each of the two longitudinal edge portions has fastening elements provided on at least a region of the first major surface, wherein the central portion comprises an innermost central region, the first major surface of the innermost central region being free of fastening elements, and wherein the second major surface is free of adhesive.

Due to the improved roll stability, the provision of twin rolls having longer roll lengths (and thus higher roll diameters) and/or more narrow cross-widths is advantageously possible. Furthermore by providing the user's ends with the fastening elements in the two outer edge regions at or close to the edges of the elongated material, it advantageously possible to provide, in addition to planetary rolls having higher stability, rolls where the elongated material is cross wound (cross wound rolls) and thereby provide wider rolls with longer roll lengths than that that can be achieved with a planetary roll of the same roll diameter.

In addition, by advantageously positioning the manufacturer's ends in the middle, the width of the manufacturer's end can be easily lengthened as needed and/or desired without immediately, detrimentally impacting roll stability. In this regard, it is to be appreciated that the use of a longer manufacturer's end and thus overall longer fastening tab in conjunction with a landing zone on e.g. a diaper or incontinence brief can be useful in providing a wider range of sizing.

As indicated above the width of the innermost central region in the central portion may be up to 30% of the total width of the elongated material, in particular ranging from about 7% to about 30% (inclusive) of the total width of the elongated material.

Preferably, the width of each longitudinal edge portion is at least 13% of the total width of the elongated material.

Each of the longitudinal edge portions may comprise an outermost edge region whose first major surface is free of fastening elements. Once a fastening tab has been cut from the roll of elongated material, such an outermost edge region provides a so-called finger lift to the user. The width of each of the outermost edge regions is preferably in the range between 3% and 10% (inclusive) of the total width of the elongated material. Preferably, the width of each of the edge regions is in the range between 3 mm and 12 mm (inclusive), more preferably between 5 mm and 9 mm (inclusive).

Preferably, each of the longitudinal edge portions comprises a fastening region with the fastening elements of the longitudinal edge portions being provided on the first major surface of each of said fastening regions. The width of each of the fastening regions is preferably in the range between 3% and 30% (inclusive), more preferably between 10% and 27% (inclusive) of the total width of the elongated material. Preferably, the width of each of the fastening regions is in the range between 10 mm and 30 mm (inclusive), more preferably between 13 mm and 25 mm (inclusive).

It is preferred that the first major surface in the entire central portion is free of fastening elements.

Preferably, the elongated material is a laminate comprising a carrier web extending over substantially the total width of the elongated material and a fastening material comprising a plurality of fastening elements. Preferably, each longitudinal edge portion is provided with at least one elongate strip of the fastening material. The at least one elongate strip of the fastening material in each longitudinal edge portion may be directly or indirectly affixed to the carrier web via, e.g., adhesive, ultrasonic welding, and/or hot-air welding or in-situ lamination (e.g. where the elongate strip of the fastening material is extruded directly onto the carrier). The carrier of the laminate preferably comprises a nonwoven. In addition, or alternatively, the fastening elements may comprise male fastening elements, which are preferably selected from the group consisting of hook fasteners, mushroom-shaped fasteners, stem-shaped fasteners, cup-shaped fasteners, T-shaped fasteners and mixtures thereof.

Twin rolls described herein are particular suited for in line processing towards affixing tabs onto absorbent articles or precursor materials for absorbent articles. Moreover while the first major surface of the innermost central region of the central portion of the rolled elongated material may be provided with adhesive, it is favorable to provide rolls wherein the first major surface of the innermost central region of the central portion is free of adhesive. This is advantageous in that such embodiments would not require a polycoat, and more particularly the means used for affixing the manufacturer's end can be selected as desired and/or needed at the time of the manufacture fastening tabs and their attachment to absorbent articles or precursor materials. It is to be appreciated that the first major surface of the central portion outside the innermost central region, in particular just adjacent to the two longitudinal edge portions, may include adhesive to provide for fastening tabs including a so-called "anti-flagging feature".

Another aspect of the present invention is a method of manufacturing fastening tabs, the method comprising the following steps:
a) providing a roll of an elongated material for manufacture of fastening tabs as described herein;
b) unwinding elongated material from the roll;
c) slitting the elongated material along its length through the innermost central region of the central portion in order to provide two elongated strips, each strip comprising one of the two longitudinal edge portions and a section of the central portion; and
d) cutting each of the two elongated strips in the width direction in order to provide fastening tabs having a user's end and a manufacturer's end.

Due to the favorable placement of the user's ends with the fastening elements in the two outer longitudinal portions of the roll, after slitting and cutting the pair of tabs generated are in correct orientation to one another (e.g. right-hand tab on the right and the left-hand one on the left) for attachment to an absorbent article such as a diaper or an incontinence brief. With prior art twin rolls with the user's ends in the middle, after slitting and cutting, the generated pair of tabs need to be either flipped or rotated to bring them in the right orientation to one another for attachment to an absorbent article such as a diaper or an incontinence brief. Accordingly twin rolls and such methods described herein are also advantageous in that in-line-handling is easier.

Methods described herein preferably comprise the step of affixing at least one of the fastening tabs at its manufacturer's end to an absorbent article or to an absorbent article precursor material. The step of affixing may comprise one or a combination of ultrasound welding, hot-air welding and adhesive bonding. The step of affixing is preferably performed essentially at the same time or after the step of cutting each of the two elongated strips in the cross direction.

Preferably, the method comprises the step of applying an adhesive onto the first major surface of at least a part of the innermost central region of the central portion between unwinding and slitting the elongated material along its length through the innermost central region of the central portion and cutting each of the two elongated strips in the cross direction. Adhesive may be applied to the entire innermost central region or just a part (e.g. a major part) of the innermost central region. For those embodiments where adhesive applied only to a part of the innermost central region, desirably the adhesive is applied such that the step of slitting the elongated material along its length through the innermost central region of the central portion comprises slitting through the adhesive provided on the first major surface of the innermost central region. In this manner, each of the two sections of the central portion obtained after slitting has adhesive in the region along and adjacent to the cut edge. The method may further comprise the step of affixing at least one of the fastening tabs at its manufacturer's end to an absorbent article or to an absorbent article precursor material using the adhesive applied onto the first major surface of the innermost central region of the central portion. Preferably, the step of affixing is performed essentially at the same time or after the step of cutting each of the two elongated strips in the cross direction.

In those embodiments where adhesive is applied onto the first major surface of the innermost central region of the central portion and the elongated material is slit along its length through said applied adhesive, the respective portion of the manufacturer's end of the final fastening tab is fully covered with adhesive all the way to its cut edge. Thus, the manufacturer's end of the final fastening tab will nicely adhere to the absorbent article or the absorbent article precursor without any loose edges or flaps protruding from the absorbent article or the absorbent article precursor material.

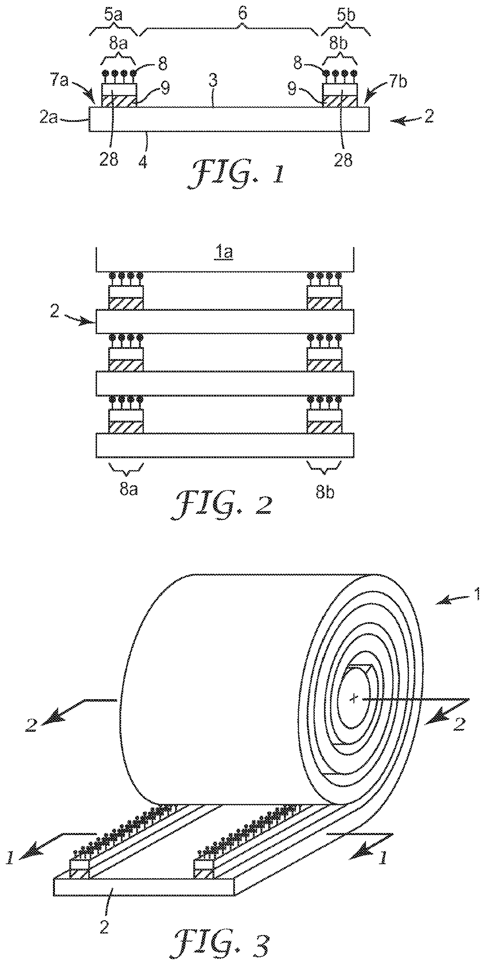

Further preferred features are illustrated with reference to preferred embodiments which are shown in the following Figures, which show:
- Fig.: 1 a schematic cross-section of an exemplary embodiment of an elongated material wound in an exemplary roll in accordance to the present invention, said exemplary roll shown in Figures 2 and 3;
- Fig. 2: a schematic, partial cross-section of an exemplary embodiment of a roll of an elongated material according to the present invention;
- Fig. 3: a schematic, perspective view of an exemplary embodiment of a roll of an elongated material according to the present invention;
- Fig. 4: a schematic, partial cross-section of another exemplary embodiment of a roll of an elongated material according to the present invention;
- Fig. 5: a schematic perspective view depicting an exemplary embodiment of a method of manufacturing fastening tabs according to the present invention;
- Fig. 6: a schematic cross-section of a fastening tab manufactured according to the present invention attached to a diaper web; and
- Fig. 7: a schematic cross-section of a further exemplary embodiment of an elongated material to be provided in a roll according to the present invention.

Figure 3 shows a schematic perspective view of an exemplary embodiment of a roll 1 of an elongated material 2 according to the present invention while Figure 1 shows a schematic cross-section of a single layer of the elongated material and Figure 2 shows a schematic, partial cross-section through a number of layers of the elongated material arranged on the roll. As can be appreciated from Figure 3, the exemplary roll is a planetary roll.

Referring to Figure 1, the elongated material 2 includes a carrier web 2a extending over substantially the total width of the elongated material and comprises a first major surface 3 and a second major surface 4, two longitudinal edge portions 5a and 5b and a central portion 6 provided in the width direction in between the longitudinal edge portions 5a and 5b. Each of the two longitudinal edge portions 5a and 5b has mechanical fastening elements 8 provided on at least a region of the first major surface 3. The width of each longitudinal edge portion 5a, 5b is at most 30% of the total width of the elongated material 2. As also shown in the exemplary embodiment, favorably each of the longitudinal edge portions 5a and 5b comprises an outermost edge region 7a and 7b, respectively, whose first major surface 3 is free of fastening elements. These outermost edge regions 7a and 7b may be used as a finger lift in the final fastening tab. In the exemplary embodiment, the remaining regions of the longitudinal edge portions 5a and 5b form fastening regions 8a and 8b, respectively, each comprising fastening elements 8. As shown in the exemplary embodiment, said fastening regions 8a and 8b may be provided with elongate strips of a fastening material 28 along the length of the elongated material, said strips comprising a plurality of fastening elements 8 as can be recognized in Figure 3. In particular, it can be recognized from Figure 3 the elongated material includes two elongate strips of fastening material extending along the longitudinal direction of the elongated material. In the exemplary embodiment shown in Figures 1 to 3, the strips of fastening material 28 are attached to the carrier web 2a via adhesive 9.

Elongate strips of the fastening material may be directly or indirectly attached via adhesive, ultrasonic welding, and/or hot air welding. In addition it will be appreciated that fastening elements may also be provided in different patterns. For example each of the fastening regions may be provided with two or more (e.g. four) separate elongate strips of fastening material, each running along the length of the elongated material so that the resulting tabs include two or more patches of fastening elements with areas free of fastening elements in between the patches. Alternatively each of the fastening regions may be provided with at least one elongate strip of fastening material that is provided in the form of a reticulated mechanical fastener comprising multiple strands of fastening materials attached and/or integral to each other at bridging regions of fastening material. Examples of such a reticulated mechanical fastener are described in detail in WO 2012/112768 A1. Specific reference is made to this document, which is incorporated by reference herein in its entirety, in particular with regard to the structure of such reticulated mechanical fasteners and the manufacturing thereof..

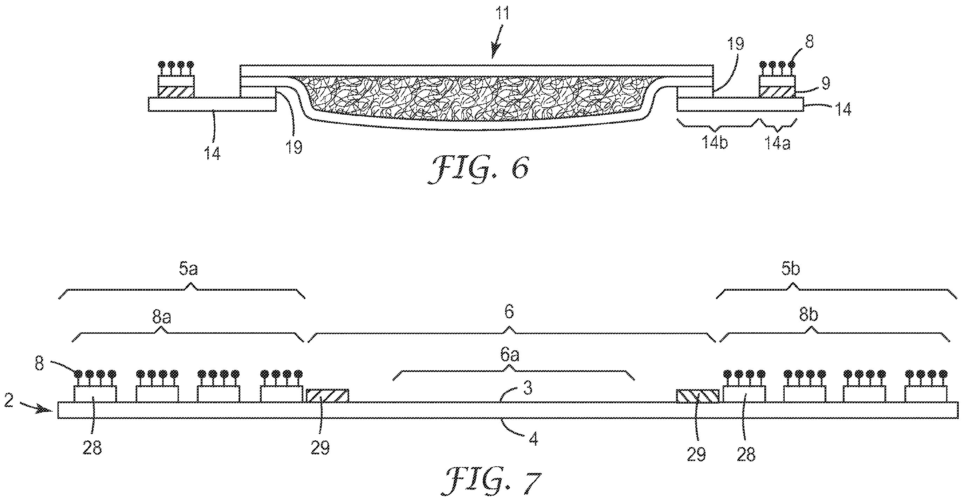

This is best understood by reference to Figure 7 showing a schematic cross-section of another exemplary embodiment of an elongated material to be provided in a roll according to the present invention. The exemplary embodiment shown in Figure 7 includes an elongated material 2 comprising a first major surface 3 and a second major surface 4, two longitudinal edge portions 5a and 5b and a central portion 6 provided in the width direction in between the longitudinal edge portions 5a and 5b and including a innermost central region 6a. Each of the two longitudinal edge portions 5a and 5b includes fastening regions 8a and 8b comprising either multiple elongate strips of fastening material with mechanical fastening elements 8 or one elongate strip of fastening material that is provided in the form of a reticulated mechanical fastener comprising multiple strands of fastening materials attached and/or integral to each other at bridging regions of fastening material. In this embodiment, the elongate strips of fastening material 28 are attached directly to the carrier web 2a via e.g. ultrasonic welding and/or hot air welding.

The fastening elements may comprise male fastening elements which are, e.g., selected from the group consisting of hook fasteners, mushroom-shaped fasteners, stem-shaped fasteners, cup-shaped fasteners, T-shaped fasteners and mixtures thereof.

Carrier webs may be single-layered or multi-layered. They may comprise a film, a nonwoven, a textile (e.g. woven or knitted), a foamed material or combinations thereof, e.g. in the form a laminate (such as multilayer film laminate, multilayer nonwoven laminate or a film-nonwoven laminate). The layer(s) of carrier webs may made of various substances such as, for example, polypropylene, polyvinylchloride, polyethylene terephthalate, polyethylene, polyolefin copolymers or blends of polyolefins such as, for example, a blend of polypropylene, low density polyethylene and/or linear low density polyethylene. Carrier webs are desirably between 30 microns and 500 microns in thickness, and more desirably between 40 and 150 microns.

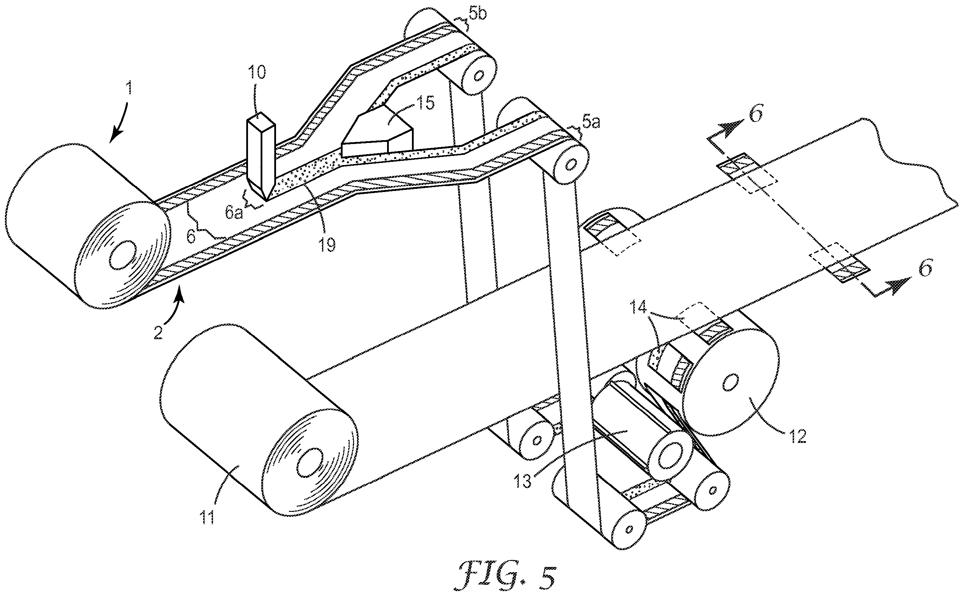

Returning to Figures 1 to 3, it can be seen that in the exemplary embodiment depicted in Figures 1 to 3, the first major surface 3 of the entire central portion 6 is free of fastening elements. As mentioned above, at least the innermost central region of the central portion is free of fastening elements so that such portion or at least a part of such portion thereof can be used to affix tab(s) obtained from such roll to absorbent article(s) or precursor materials. This can be readily appreciated from Figure 5 which shows a schematic perspective view depicting an exemplary embodiment of a method of manufacturing fastening tabs where adhesive is applied onto the innermost central region 6a of the central portion 6. The method will be discussed in more detail below. In the exemplary embodiment shown in Figures 1 to 3, it can be seen that the first major surface 3 across the entire width of the central portion 6 is also free of adhesive.

As mentioned above in favorable embodiments, the innermost central region is free of adhesive. In addition while it may be desirable that the first major surface of the entire central portion is free of fastening elements, the first major surface of the central portion in regions outside the innermost central region, in particular just adjacent to the two longitudinal edge portions, may include adhesive for example to provide for fastening tabs including a so-called "anti-flagging feature". This is best understood by reference to the exemplary embodiment shown in Figure 7. In particular in exemplary elongated material shown in Figure 7 the first major surface 3 across the entire central portion 6 is free of fastening elements. In addition, while the first major surface 3 across the innermost central region 6a is free of adhesive, it can be seen that the first major surface of the central portion in two side regions outside the innermost central region, in particular just adjacent to the two longitudinal edge portions, may be provided with an adhesive 29 to provide for an "anti-flagging feature" in the resulting fastening tabs.

Returning to the exemplary embodiment shown in Figures 1 to 3, in particular to the illustration of Figure 2, the elongated material 2 is wound up on a roll 1, in the form of a planetary roll, e.g. comprising a roll core 1a. Since the thickness of the elongated material 2 is largest in the fastening regions 8a and 8b, which comprise the fastening elements 8, contact between adjacent layers of the elongated material 2 being wound up on a roll 1 is, in essence, provided in these fastening regions 8a and 8b. Since these fastening regions 8a and 8b are provided at or close to the side edges of the elongated material 2 and thus the roll 1, the roll reveals enhanced stability, in particular during unwinding and handling. Furthermore, penetration of dust and dirt particles is effectively prohibited and/or reduced due to the presence of at the fastening regions 8a and 8b at or close to the edges of the roll.

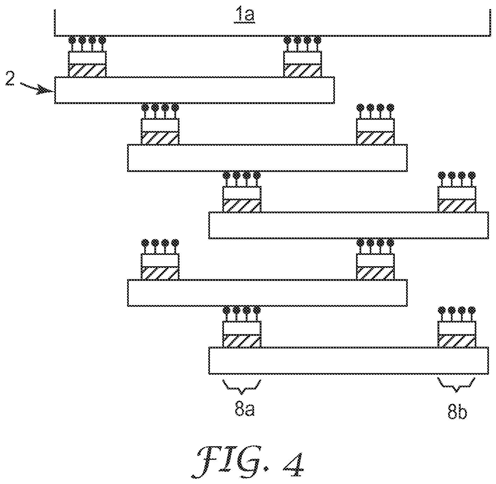

As mentioned above, the roll may also favorably be provided in the form of a cross wound roll. Figure 4 shows a schematic, partial cross-section of such a roll. The elongated material 2 of the embodiment shown in Figure 4 is the same as that shown in Figures 1 to 3. As can be appreciated from a comparison of Figures 2 and Figure 4, instead of winding the elongated material directly on top of itself, the elongated material is wound helically back and forth across the width of the roll, in particular onto a roll core 1a. It will be appreciated from Figure 4, the positioning of the fastening regions 8a and 8b at or close to the side edges of the elongated material 2 desirably allows for a transverse offset in the next winding of elongated material (and thus crossing winding of the elongated material), since at least one of fastening regions of the next winding can be positioned between those of the previous winding. It will also be appreciated from a comparison of Figures 2 and 4 that the cross wound roll is wider than the planetary roll, which further facilitates roll stability. In addition as mentioned above, for a given roll diameter, the provision of a cross wound roll allows for longer roll lengths relative to a planetary roll.

Figure 5 schematically shows an exemplary embodiment of a method of manufacturing fastening tabs and of fixing said fastening tabs at their manufacturer's end to an absorbent article or to an absorbent article precursor material. In particular, Figure 5 depicts the manufacture of fastening tabs and the affixing of the generated fastening tabs to an absorbent article precursor material 11, which may be a diaper or incontinence brief precursor web. As can be seen, a roll 1 of an elongated material 2, such as the roll shown in Figure 3, is provided. The elongated material 2 is unwound from the roll 1 and an adhesive 19 is applied onto the first major surface 3 of the innermost central region 6a of the central portion 6 by an adhesive applicator head 10. The elongated material 2 is then slit along its length through the innermost central region 6a by means of a cutting tool 15 which also slits through the adhesive 19 provided on the first major surface 3 of the innermost central region. Thus, the elongated material 2 is divided into two endless sheets or strips of tab material, which are essentially mirror images of one another. Each of the two elongated strips of tab material comprises one of the two longitudinal edge portions 5a and 5b and a section of the central portion 6. Each of the two elongated strips are then cut in the cross direction in order to provide fastening tabs 14, each having a user's end and a manufacturer's end. In the exemplary embodiment shown in Figure 5, each of the two elongated strips of fastening tab material is fixed to a vacuum wheel 12 and cut by a cutting wheel 13 into individual fastening tabs 14 while being kept in place on the vacuum wheel 12. The individually cut fastening tabs 14 are kept in place by the vacuum wheel 12 until they are applied to the diaper or incontinence brief precursor web 11. As shown in Figure 5, as the web 11 moves forward (from left to right in the Figure) the fastening tabs 14 are affixed pair-wise at an appropriate interval to the web 11 using the adhesive 19 applied previously onto the first major surface 3 of the innermost central region of the central portion 6. As is evident from Figure 5, the provision of the user's ends of the fastening tabs to be cut from the elongated material in the outer edge portions of the elongated material also allows for an easier application of the cut fastening tabs to a web, because the cut fastening tabs may be applied directly to the web in their orientation as cut without having to be flipped or rotated by 180°. Figure 6 shows a schematic cross-section through the diaper or incontinence brief precursor web 11 with the fastening tabs 14 being affixed to the web via the adhesive 19 in the manufacturer's ends 14b with the user's ends 14a extending outwardly away from the web. As can be appreciated from Figure 6, since the adhesive 19 is provided to the very edge of the tab in the manufacturer's end 14b, the edge of the tab is cleanly adhered to the web with no remaining flap of non-adherent tab material.

It is to be mentioned that although not as favorable as the exemplary method shown in Figure 5, one could alternatively apply adhesive after the slitting step and prior to cutting step, i.e. apply adhesive at or close to each of the innermost edge regions of the two elongated strips of fastening tab material generated upon slitting.

It will be appreciated that the fastening tabs may be attached to absorbent articles per se or to alternative absorbent article precursor materials. In regard to the latter, for example, fastening tabs 14 may be instead, affixed to an ear web, said ear web may, in turn, be affixed to a diaper or incontinence brief precursor web.

The fastening tabs may also be affixed to the absorbent articles or absorbent article precursor materials by other techniques such as ultrasound welding, hot-air welding or the like. In such cases, one would dispense with the step of applying adhesive onto the first major surface of the elongated material e.g. as shown in Figure 5.

## Claims

1. Roll (1) of an elongated material (2) for manufacture of fastening tabs, the elongated material (2) comprising first (3) and second (4) major surfaces, two longitudinal edge portions (5a, 5b) and a central portion (6) provided in the width direction in between the two longitudinal edge portions (5a, 5b),
- the width of each longitudinal edge portion (5a, 5b) being at most 30% of the total width of the elongated material (2), wherein
- each of the two longitudinal edge portions (5a, 5b) has mechanical fastening elements (8) provided on at least a region of the first major surface (3), wherein
- the central portion (6) comprises an innermost central region (6a), the first major surface (3) of the innermost central region being free of fastening elements, and wherein
- the second major surface (4) is free of adhesive.

2. The roll of claim 1, wherein the first major surface (3) of the innermost central region of the central portion (6) is free of adhesive.

3. The roll of claim 1 or 2, wherein the width of the innermost central region is up to 30% of the total width of the elongated material, in particular ranges from about 7% to about 30% (inclusive) of the total width of the elongated material; and/or wherein the width of each longitudinal edge portion (5a, 5b) is at least 13% of the total width of the elongated material (2).

4. The roll of any of the previous claims, wherein each of the longitudinal edge portions (5a, 5b) comprises an outermost edge region (7a, 7b) whose first major surface (3) is free of fastening elements.

5. The roll of claim 4, wherein the width of each of the outermost edge regions (7a, 7b) is in the range between 3% and 10% (inclusive) of the total width of the elongated material (2) and/or wherein the width of each of the outermost edge regions (7a, 7b) is in the range between 3 mm and 12 mm (inclusive), preferably between 5 mm and 9 mm (inclusive).

6. The roll of any of the previous claims, wherein each of the longitudinal edge portions (5a, 5b) comprises a fastening region (8a, 8b), the fastening elements (8) being provided on the first major surface (3) of each of said fastening regions (8a, 8b).

7. The roll of claim 6, wherein the width of each of the fastening regions (8a, 8b) is in the range between 3% and 30% (inclusive), in particular 10% to 27% (inclusive) of the total width of the elongated material (2) and/or wherein the width of each of the fastening regions (8a, 8b) is in the range between 10 mm and 30 mm (inclusive), preferably between 13 mm and 25 mm (inclusive).

8. The roll of any of the previous claims, wherein the elongated material (2) is a laminate comprising a carrier web (2a) extending over substantially the total width of the elongated material and a fastening material (28) comprising a plurality of fastening elements (8), wherein each longitudinal edge portion (5a, 5b) is provided with at least one elongate strip of the fastening material.

9. The roll of claim 8, wherein the at least one elongate strip of the fastening material (28) in each longitudinal edge portion is directly or indirectly affixed to the carrier web, in particular via adhesive (9), ultrasonic welding, and/or hot air welding or via in-situ lamination; and/or wherein the carrier web comprises a material selected from the group consisting of films, non-wovens, textiles, foams and combinations thereof.

10. The roll of any of the previous claims, wherein the fastening elements (8) comprise male fastening elements, in particular male fastening elements selected from the group consisting of hook fasteners, mushroom-shaped fasteners, stem-shaped fasteners, cup-shaped fasteners, T-shaped fasteners and mixtures thereof.

11. The roll of any of the previous claims, wherein the roll is a planetary roll or a cross wound roll.

12. A method of manufacturing fastening tabs (14), the method comprising the following steps:
a) providing a roll (1) of an elongated material for manufacture of fastening tabs (2) according to any of the previous claims;
b) unwinding elongated material from the roll (1);
c) slitting the elongated material (2) along its length through the innermost central region (6a) of the central portion (6) in order to provide two elongated strips, each strip comprising one of the two longitudinal edge portions and a section of the central portion; and
d) cutting each of the two elongated strips in the width direction in order to provide fastening tabs (14) having a user's end (14a) and a manufacturer's end (14b).

13. The method of claim 12, further comprising the step of affixing at least one of the fastening tabs (14) at its manufacturer's end (14b) to an absorbent article or to an absorbent article precursor material (11), in particular by means of one or a combination of: ultrasound welding, hot-air welding and adhesive bonding, wherein the step of affixing is performed essentially at the same time or after the step d) of cutting.

14. The method of claim 12, further comprising the step of applying an adhesive (19) onto the first major surface (3) of at least a part of the innermost central region (6a) of the central portion (6) between steps b) and c).

15. The method of claim 14, wherein step c) comprises slitting through the adhesive (19) provided on the first major surface (3) of the innermost central region.

16. The method of claim 14 or 15, further comprising the step of affixing at least one of the fastening tabs (14) at its manufacturer's end (14b) to an absorbent article or to an absorbent article precursor material (11, 111) using the adhesive (19), wherein the step of affixing is performed essentially at the same time or after the step d) of cutting.

17. The method of claim 13 or 16 wherein the absorbent article is a diaper or incontinence brief; and/or wherein the absorbent article precursor material is a diaper precursor web, incontinence brief precursor web or a ear web.

## Patentansprüche

1. Eine Rolle (1) eines länglichen Materials (2) zur Herstellung von Befestigungslaschen, wobei das längliche Material (2) eine erste (3) und ein zweite (4) Hauptoberfläche, zwei Längskantenabschnitte (5a, 5b) und einen Mittelteil (6), der in Breitenrichtung zwischen den zwei Längskantenabschnitten (5a, 5b) bereitgestellt ist, umfasst,
- wobei die Breite jedes Längskantenabschnitts (5a, 5b) höchstens 30 % der Gesamtbreite des länglichen Materials (2) beträgt, wobei
- jeder der zwei Längskantenabschnitte (5a, 5b) mechanische Befestigungselemente (8) aufweist, die auf mindestens einem Bereich der ersten Hauptoberfläche (3) bereitgestellt sind, wobei
- der Mittelteil (6) einen innersten Mittelbereich (6a) umfasst, wobei die erste Hauptoberfläche (3) des innersten Mittelbereichs frei von Befestigungselementen ist, und wobei
- die zweite Hauptoberfläche (4) frei von Klebstoff ist.

2. Die Rolle nach Anspruch 1, wobei die erste Hauptoberfläche (3) des innersten Mittelbereichs des Mittelteils (6) keinen Klebstoff aufweist.

3. Die Rolle nach Anspruch 1 oder 2, wobei die Breite des innersten Mittelbereichs bis zu 30 % der Gesamtbreite des länglichen Materials, insbesondere im Bereich von etwa 7 % bis (einschließlich) etwa 30 % der Gesamtbreite des länglichen Materials, beträgt; und/oder wobei die Breite jedes Längskantenabschnitts (5a, 5b) mindestens 13 % der Gesamtbreite des länglichen Materials (2) beträgt.

4. Die Rolle nach einem der vorstehenden Ansprüche, wobei jeder der Längskantenabschnitte (5a, 5b) einen äußersten Kantenbereich (7a, 7b) umfasst, dessen erste Hauptoberfläche (3) keine Befestigungselemente aufweist.

5. Die Rolle nach Anspruch 4, wobei die Breite eines jeden der äußersten Kantenbereiche (7a, 7b) einen Bereich zwischen 3 % und (einschließlich) 10 % der Gesamtbreite des länglichen Materials (2) beträgt und/oder wobei die Breite jeder der äußersten Kantenbereiche (7a, 7b) einen Bereich von 3 mm bis (einschließlich) 12 mm, vorzugsweise jedoch von 5 mm bis (einschließlich) 9 mm beträgt.

6. Die Rolle nach einem der vorherigen Ansprüche, wobei jeder der Längskantenabschnitte (5a, 5b) einen Befestigungsbereich (8a, 8b) umfasst, wobei die Befestigungselemente (8) sich auf der ersten Hauptoberfläche (3) eines jeden der Befestigungsbereiche (8a, 8b) befinden.

7. Die Rolle nach Anspruch 6, wobei die Breite eines jeden der Befestigungsbereiche (8a, 8b) sich in einem Bereich zwischen 3 % und (einschließlich) 30 % (im Besonderen zwischen 10 % und (einschließlich) 27 % der Gesamtbreite des länglichen Materials (2), und/oder wobei die Breite eines jeden der Befestigungsbereiche (8a, 8b) sich in einem Bereich zwischen 10 mm und (einschließlich) 30 mm, vorzugsweise zwischen 13 mm und (einschließlich) 25 mm befindet.

8. Die Rolle nach einem der vorstehenden Ansprüche, wobei das längliche Material (2) als Schichtstoff vorhanden ist, der ein Trägerband (2a) umfasst, welches sich über die Gesamtbreite des länglichen Materials und eines Befestigungsmaterials (28), welches eine Vielzahl von Befestigungselementen (8) enthält, erstreckt, wobei jeder Längskantenabschnitt (5a, 5b) mit mindestens einem länglichen Streifen des Befestigungsmaterials bereitgestellt ist.

9. Die Rolle nach Anspruch 8, wobei der mindestens eine längliche Streifen des Befestigungsmaterials (28) in jedem Längskantenabschnitt direkt oder indirekt an der Trägerbahn befestigt ist, insbesondere durch Klebstoff (9), Ultraschallschweißen und/oder Heißluftschweißen oder durch In-situ-Lamination; und/oder wobei die Trägerbahn ein Material umfasst, das aus der Gruppe ausgewählt ist, die aus Folien, Vliesstoffen, Textilien, Schäumen und Kombinationen davon besteht.

10. Die Rolle nach einem der vorstehenden Ansprüche, wobei die Befestigungselemente (8) Befestigungselemente mit Außenstecker, insbesondere ausgewählt aus der Gruppe bestehend aus Häkchenverschlüssen, pilzförmigen, stielförmigen, becherförmigen, T-förmigen Befestigungselementen und Mischungen davon umfassen.

11. Die Rolle nach einem der vorstehenden Ansprüche, wobei die Rolle eine Planetenrolle oder eine Kreuzwickelrolle ist.

12. Ein Verfahren zum Herstellen von Befestigungslaschen (14), wobei das Verfahren die folgenden Schritte umfasst:
a) Bereitstellen einer Rolle (1) eines länglichen Materials (2) zum Herstellen von Befestigungslaschen (2) nach einem der vorstehenden Ansprüche;
b) Abrollen des länglichen Materials von der Rolle (1);
c) Schlitzen des länglichen Materials (2) entlang seiner Länge durch den innersten Mittelbereich (6a) des Mittelteils (6), um zwei längliche Streifen bereitzustellen, die jeweils einen der beiden Längskantenabschnitte und einen Teilabschnitt des Mittelteils umfassen; und
d) Schneiden jedes der beiden länglichen Streifen, um Befestigungslaschen (14) mit einem Benutzer-Ende (14a) und einem Hersteller-Ende (14b) bereitzustellen.

13. Das Verfahren nach Anspruch 12, das ferner den Schritt umfasst, dass mindestens eine der Befestigungslaschen (14) am Hersteller-Ende (14b) an einem Absorptionsartikel oder ein Absorptionsartikel-Vorläufermaterial (11) angebracht wird, insbesondere durch eines von oder eine Kombination aus: Ultraschallverschweißung, Heißluftverschweißung und Klebehaftung, wobei der Schritt der Anhaftung im Wesentlichen gleichzeitig mit oder nach dem Schritt d) des Schneidens erfolgt.

14. Das Verfahren nach Anspruch 12, das ferner den Schritt umfasst, dass zwischen den Schritten b) und c) ein Klebstoff (19) auf die erste Hauptoberfläche (3) mindestens eines Teils des innersten Mittelbereichs (6a) des Mittelteils (6) angebracht wird.

15. Das Verfahren nach Anspruch 14, wobei Schritt c) das Schlitzen durch den Klebstoff (19) umfasst, der auf der ersten Hauptoberfläche (3) des innersten Mittelbereichs bereitgestellt ist.

16. Verfahren nach Anspruch 14 oder 15, das ferner den Schritt der Anhaftung mindestens einer der Befestigungslaschen (14) an ihrem Hersteller-Ende (14b) an einen Absorptionsartikel oder ein Absorptionsartikel-Vorläufermaterial (11, 111) umfasst, unter Anwendung des Klebstoffs (19), wobei der Schritt der Anhaftung im Wesentlichen gleichzeitig mit oder nach dem Schritt d) des Schneidens erfolgt.

17. Das Verfahren nach Anspruch 13 oder 16, wobei der Absorptionsartikel eine Windel oder eine Inkontinenzhose ist; und/oder wobei das Absorptionsartikel-Vorläufermaterial eine Windelvorläuferbahn, eine Inkontinenzhose-Vorläuferbahn oder eine Ohrbahn ist.

## Revendications

1. Rouleau (1) d'un matériau allongé (2) pour la fabrication de languettes de fixation, le matériau allongé (2) comprenant des première (3) et deuxième (4) surfaces principales, deux parties de bord longitudinal (5a, 5b) et une partie centrale (6) fournie dans la direction en largeur entre les deux parties de bord longitudinal (5a, 5b),
- la largeur de chaque partie de bord longitudinal (5a, 5b) valant au plus 30 % de la largeur totale du matériau allongé (2), dans lequel
- chacune des deux parties de bord longitudinal (5a, 5b) a des éléments de fixation mécanique (8) fournis sur au moins une région de la première surface principale (3), dans lequel
- la partie centrale (6) comprend une région centrale interne (6a), la première surface principale (3) de la région centrale interne étant dépourvue d'éléments de fixation, et dans lequel
- la deuxième surface principale (4) est dépourvue d'adhésif.

2. Rouleau selon la revendication 1, dans lequel la première surface principale (3) de la région centrale interne de la partie centrale (6) est dépourvue d'adhésif.

3. Rouleau selon la revendication 1 ou 2, dans lequel la largeur de la région centrale interne va jusqu'à 30 % de la largeur totale du matériau allongé, en particulier va d'environ 7 % à environ 30 % (inclus) de la largeur totale du matériau allongé ; et/ou dans lequel la largeur de chaque partie de bord longitudinal (5a, 5b) vaut au moins 13 % de la largeur totale du matériau allongé (2).

4. Rouleau selon l'une quelconque des revendications précédentes, dans lequel chacune des parties de bord longitudinal (5a, 5b) comprend une région de bord externe (7a, 7b) dont la première surface principale (3) est dépourvue d'éléments de fixation.

5. Rouleau selon la revendication 4, dans lequel la largeur de chacune des régions de bord externes (7a, 7b) est dans la plage entre 3 % et 10 % (inclus) de la largeur totale du matériau allongé (2) et/ou dans lequel la largeur de chacune des régions de bord externes (7a, 7b) est dans la plage entre 3 mm et 12 mm (inclus), de préférence entre 5 mm et 9 mm (inclus).

6. Rouleau selon l'une quelconque des revendications précédentes, dans lequel chacune des parties de bord longitudinal (5a, 5b) comprend une région de fixation (8a, 8b), les éléments de fixation (8) étant fournis sur la première surface principale (3) de chacune desdites régions de fixation (8a, 8b).

7. Rouleau selon la revendication 6, dans lequel la largeur de chacune des régions de fixation (8a, 8b) est dans la plage entre 3 % et 30 % (inclus), en particulier 10 % à 27 % (inclus) de la largeur totale du matériau allongé (2) et/ou dans lequel la largeur de chacune des régions de fixation (8a, 8b) est dans la plage entre 10 mm et 30 mm (inclus), de préférence entre 13 mm et 25 mm (inclus).

8. Rouleau selon l'une quelconque des revendications précédentes, dans lequel le matériau allongé (2) est un stratifié comprenant une bande de support (2a) s'étendant essentiellement sur la largeur totale du matériau allongé et un matériau de fixation (28) comprenant une pluralité d'éléments de fixation (8), dans lequel chaque partie de bord longitudinal (5a, 5b) est pourvue d'au moins une bande allongée du matériau de fixation.

9. Rouleau selon la revendication 8, dans lequel ladite au moins une bande allongée du matériau de fixation (28) dans chaque partie de bord longitudinal est directement ou indirectement attachée à la bande de support, en particulier par l'intermédiaire d'un adhésif (9), d'un soudage par ultrasons et/ou de soudage par air chaud ou par l'intermédiaire d'une stratification in situ ; et/ou dans lequel la bande de support comprend un matériau choisi dans le groupe constitué de films, non-tissés, textiles, mousses et des combinaisons de ceux-ci.

10. Rouleau selon l'une quelconque des revendications précédentes, dans lequel les éléments de fixation (8) comprennent des éléments de fixation mâles, en particulier des éléments de fixation mâles choisis dans le groupe constitué de fermoirs à crochet, fermoirs en forme de champignon, fermoirs en forme de tige, fermoirs en forme de coupelle, fermoirs en forme de T et des mélanges de ceux-ci.

11. Rouleau selon l'une quelconque des revendications précédentes, où le rouleau est un rouleau planétaire ou un rouleau à enroulement croisé.

12. Procédé de fabrication de languettes de fixation (14), le procédé comprenant les étapes suivantes :
a) fourniture d'un rouleau (1) d'un matériau allongé pour la fabrication de languettes de fixation (2) selon l'une quelconque des revendications précédentes ;
b) déroulement du matériau allongé du rouleau (1) ;
c) refendage du matériau allongé (2) sur sa longueur à travers la région centrale interne (6a) de la partie centrale (6) afin de fournir deux bandes allongées, chaque bande comprenant une des deux parties de bord longitudinal et une section de la partie centrale ; et
d) découpe de chacune des deux bandes allongées dans la direction en largeur afin de fournir des languettes de fixation (14) ayant un extrémité de l'utilisateur (14a) et une extrémité du fabricant (14b).

13. Procédé selon la revendication 12, comprenant en outre l'étape consistant à apposer au moins une des languettes de fixation (14) au niveau de son extrémité du fabricant (14b) sur un article absorbant ou sur un matériau précurseur d'article absorbant (11), en particulier au moyen d'un ou d'une combinaison parmi : soudage par ultrasons, soudage à air chaud et liaison adhésive, dans lequel l'étape d'apposition est mise en œuvre sensiblement en même temps que, ou après l'étape d) de découpe.

14. Procédé selon la revendication 12, comprenant en outre l'étape consistant à appliquer un adhésif (19) sur la première surface principale (3) d'au moins une partie de la région centrale interne (6a) de la partie centrale (6) entre les étapes b) et c).

15. Procédé selon la revendication 14, dans lequel l'étape c) comprend un refendage à travers l'adhésif (19) fourni sur la première surface principale (3) de la région centrale interne.

16. Procédé selon la revendication 14 ou 15, comprenant en outre l'étape consistant à fixer au moins une des languettes de fixation (14) au niveau de son extrémité du fabricant (14b) sur un article absorbant ou sur un matériau précurseur d'article absorbant (11, 111) en utilisant l'adhésif (19), dans lequel l'étape de fixation est effectuée sensiblement en même temps que, ou après, l'étape d) de découpage.

17. Procédé selon la revendication 13 ou 16, dans lequel l'article absorbant est une couche ou une culotte pour l'incontinence ; et/ou dans lequel le matériau précurseur d'article absorbant est une bande de précurseur de couche, une bande de précurseur de culotte pour l'incontinence ou une bande de patte.
